# EUROPEAN PATENT APPLICATION

(11) **EP 4 527 404 A1**
(43) Date of publication of application: **26.03.2025**
(21) Application number: 22942799.2
(22) Date of filing: 16.05.2022
(51) Int. Cl.: A61K 38/44, A61K 38/16, A61P 43/00, A61P 1/00, A23L 33/195

(54) **SUPEROXIDE DISMUTASE AND USES THEREOF FOR PREVENTING OR TREATING MUCOSITIS**

(71) Applicant: Genofocus, Inc., Daejeon 34014 (KR); BiomLogic, Inc., Seoul 03923 (KR)
(72) Inventor: PAN, Jae Gu, Seoul 03923 (KR); KIM, Eui Joong, Daejeon 34014 (KR); CHANG, In Ik, Seoul 03923 (KR); KIM, Jeong Hyun, Daejeon 34014 (KR)
(74) Representative: Ullrich & Naumann PartG mbB
(86) International application number: PCT/KR2022/007002
(87) International publication number: WO 2023/224135

(57) **Abstract**

The present invention relates to superoxide dismutase, and uses thereof for preventing or treating mucositis. The superoxide dismutase according to the present invention can be effectively used for treating or improving the inhibition of a proliferation of mucous epithelium cells, which is a side effect of anticancer agents or radiotherapy.

## Description

### Technical Field

The present disclosure relates to a use of superoxide dismutase for prevention or treatment of mucositis. More specifically, the present disclosure relates to a superoxide dismutase useful for prevention or treatment of mucositis, and a use, a composition, or a method thereof for prevention or treatment of mucositis.

### Background Art

Mucositis is a pathological condition characterized by mucosal damage caused by mild inflammation to, in severe cases, ulceration. Mucositis is one of the common side effects that often occur after chemotherapy and radiotherapy. It is frequently observed in the oral cavity, but also easily occurs in the small intestine, esophagus, stomach, and large intestine. Mucositis occurs in 40% of patients receiving chemotherapy. In patients with head and neck cancer, the incidence of mucositis increases to about 90% after chemotherapy and radiotherapy. Currently, several therapeutic methods have been suggested, but their efficacy is not yet wellestablished (Oral mucositis: the hidden side of cancer therapy. J Exp Clin Cancer Res 39,210 (2020); https://www.ons.org/pep/mucositis). In the past, the most common side effects of anticancer treatment were vomiting and decreased immunity due to bone marrow hypofunction; however, recently, mucositis has been considered one of the serious side effects in the treatment of cancer patients. The epithelial cells of the oral and gastrointestinal mucosa are the fastestgrowing tissues in the body. In particular, the small intestine has the fastest turnover rate, with replacement of the epithelial cells occurring about every 7 days. Chemotherapy or radiotherapy may interfere with regeneration of mucosal cells, and malnutrition in patients may further worsen cell regeneration. Damage to the intestinal mucosa caused by radiation is believed to be attributed to microvascular damage caused by apoptosis of vascular endothelial cells. Vascular endothelial cells and platelets are presumed to play a role in the pathogenesis of mucositis.

Mucositis may cause severe pain, increased risk of systemic infection, and decreased nutritional intake in many patients. In some patients, mucositis may prevent chemotherapy from being administered at an appropriate dose, which may lead to a decline in the patients' willingness to continue treatment and may severely reduce their quality of life. To date, therapeutic agents for oral mucositis are actively studied; however, there remains a need for new methods for preventing or treating oral mucositis and gastrointestinal mucositis.

### Disclosure of Invention

### Technical Problem

The object of the present disclosure is to solve the above-mentioned problems of the prior art.

Another object of the present disclosure is to provide a superoxide dismutase for preventing or treating mucositis.

Yet another object of the present disclosure is to provide a superoxide dismutase, which is derived from Bacillus and for which oral administration efficacy and safety are secured, for preventing or treating mucositis.

Still yet another object of the present disclosure is to provide a superoxide dismutase, which is derived from *Bacillus amyloliquefaciens* and for which oral administration efficacy and safety are secured, for preventing or treating mucositis.

Still yet another object of the present disclosure is to provide a superoxide dismutase, which is derived from Bacillus or *Bacillus amyloliquefaciens* and is secreted extracellularly for convenient production, for preventing or treating mucositis.

Still yet another object of the present disclosure is to provide a pharmaceutical composition or method for preventing or treating mucositis.

Still yet another object of the present disclosure is to provide a food composition for preventing or ameliorating mucositis.

Still yet another object of the present disclosure is to provide a composition or method for preventing or treating mucositis that may occur during anticancer treatment.

The object of the present disclosure is not limited to the above-mentioned objects. The objects of the present disclosure will become clearer from the following description and may be realized by means and combinations thereof as set forth in the claims.

### Solution to Problem

Representative configurations of the present disclosure to achieve the above-mentioned objects are as follows.

According to an aspect of the present disclosure, there is provided an improved superoxide dismutase (SOD) that exhibits an effect of preventing, ameliorating, or treating mucositis.

According to another aspect of the present disclosure, there is provided a superoxide dismutase derived from a generally regarded as safe (GRAS) bacterium, such as Bacillus, for preventing, ameliorating, or treating mucositis.

According to yet another aspect of the present disclosure, there is provided a superoxide dismutase, which is derived from *Bacillus amyloliquefaciens* and for which oral administration efficacy and safety are secured, for preventing, ameliorating, or treating mucositis.

According to still yet another aspect of the present disclosure, there is provided a superoxide dismutase, which is derived from Bacillus or *Bacillus amyloliquefaciens* and is secreted extracellularly for convenient production, for preventing or treating mucositis.

According to still yet another aspect of the present disclosure, there is provided an improved superoxide dismutase that exhibits an effect of preventing, ameliorating, or treating mucositis in a subject who is undergoing radiotherapy and/or chemotherapy.

According to still yet another aspect of the present disclosure, there is provided a pharmaceutical composition for preventing or treating mucositis, comprising the superoxide dismutase as an active ingredient.

According to still yet another aspect of the present disclosure, there is provided a food or feed composition for preventing or ameliorating mucositis, comprising the superoxide dismutase as an active ingredient.

According to still yet another aspect of the present disclosure, there is provided a kit for treating or preventing mucositis, comprising a composition that comprises the superoxide dismutase as an active ingredient, and an instruction manual that comprises instructions for taking or administering the composition.

According to still yet another aspect of the present disclosure, there is provided a method for preventing, ameliorating, or treating mucositis, comprising administering to a subject the superoxide dismutase or the composition.

According to still yet another aspect of the present disclosure, there is provided a use of the superoxide dismutase for preventing or treating mucositis.

According to still yet another aspect of the present disclosure, there is provided a use of the superoxide dismutase, in manufacture of a medicament for prevention or treatment of mucositis.

### Advantageous Effects of Invention

The superoxide dismutase, composition, method, and kit according to the present disclosure can be effectively used for prevention or treatment of mucositis. In particular, it has been identified that the superoxide dismutase according to the present disclosure is effective in preventing or treating mucositis, even at low doses, in a case of being applied in the oral cavity or administered orally.

The superoxide dismutase according to the present disclosure is derived from Bacillus or *Bacillus amyloliquefaciens* which is a generally regarded as safe (GRAS) bacterium. Thus, for the superoxide dismutase, not only can oral administration efficacy and safety be secured, but also production advantage can be taken by being directly recoverable from the supernatant during culture.

According to a specific example of the present disclosure, experimental animals (rats) were used to induce mucositis, and the SOD of the present disclosure was applied at various concentrations through application in the oral cavity and oral administration, and body weight and food intake were measured. As a result, it was found that the body weight and food intake, which had decreased due to induction of mucositis, increased again in the SOD-administered group compared to the non-SOD-administered group. Furthermore, the degree of improvement in re-epithelialization and villous atrophy in oral and intestinal mucositis was observed in a SOD concentration-dependent manner, and it was found that the level of reactive oxygen species (ROS) in the gastrointestinal tract (for example, small intestine) and blood decreased in a SOD concentration-dependent manner. Therefore, the superoxide dismutase according to the present disclosure can be effectively used to prevent, ameliorate, or treat mucositis by decreasing the ROS level, inducing re-epithelialization of epithelial cells, improving atrophied villous cells, or the like, which enables normal nutrient intake, thereby allowing for a weight gain effect to be expected.

### Brief Description of Drawings

FIG. 1 illustrates a schematic diagram for the double crossover recombination performed in Example 1.1.
FIG. 2 illustrates results obtained by identifying defective genes using PCR in the expression host GFBS220 and the production strain BSBA310. W represents wild-type *B*. *subtilis* KCTC 3135, 220 represents the expression host GFBS220, and 310 represents the SodA2 production strain BSBA310.
FIG. 3 illustrates comparison of the amino acid sequences between SodA (Mn-SOD identified through N-terminal sequencing from the culture supernatant of *Bacillus amyloliquefaciens* strain GF423) and the superoxide dismutase (SodA2) of the present disclosure.
FIG. 4 illustrates an expression vector for overexpression of *sodA2* gene. Here, rrnB T1T2 represents a transcription terminator; rep(pBR322) represents a pBR322-derived replicon that operates in *E. coli*.; rep(pUB110) represents a pUB110-derived replicon that operates in *B. subtilis*; Kan^{R} represents a kanamycin resistance gene (aminoglycoside O-nucleotidyltransferase); and BJ27 promoter represents a strong promoter for *B. subtilis.*
FIG. 5 illustrates an overall procedure for preparing the production strain BSBA310.
FIGS. 6A and 6B illustrate results obtained by measuring body weight and food intake in an animal model of mucositis.
FIGS. 7A to 7C illustrate results obtained by evaluating oral mucositis in an animal model of mucositis. FIG. 7A illustrates results obtained by performing scoring of oral mucositis, FIG. 7B shows results obtained by performing histological evaluation of oral mucositis, and FIG. 7C quantitatively illustrates the thickness of re-epithelialized oral mucosal epithelium.
FIGS. 8A to 8C illustrate results obtained by evaluating intestinal mucositis in an animal model of mucositis. FIG. 8A illustrates results obtained by performing scoring of intestinal mucositis, and FIG. 8B illustrates results obtained by performing histological evaluation of intestinal mucositis. FIG. 8C quantitatively illustrates the intestinal villi length.
FIGS. 9A and 9B illustrate results obtained by performing ROS evaluation in the intestine and blood.

### Modes for Carrying out Invention

The detailed description of the present disclosure set forth below will be described with reference to specific drawings with respect to an embodiment in which the present disclosure may be practiced; however, the present disclosure is not limited thereto and, if properly described, is limited only by the appended claims, along with the full scope of equivalents to which such claims are entitled. It should be understood that various embodiments/examples of the present disclosure, although different, are not necessarily mutually exclusive. For example, a particular feature, structure, or characteristic described herein may be changed from one embodiment to another embodiment or implemented in combinations of embodiments without departing from the technical spirit and scope of the present disclosure. Unless otherwise indicated, terms used in describing the present disclosure are to be understood in their ordinary meaning and apply to the same terms used herein as well as to the aspect or embodiment of the disclosure for which the term is defined. For purposes of interpreting this specification, the following definitions will apply and whenever appropriate, terms used in the singular will also include the plural and vice versa.

### Definition

The term "subject" is used interchangeably with "patient," and may be any mammal such as primate (for example, human), companion animal (for example, dog, cat and the like), domestic animal (for example, cow, pig, horse, sheep, goat and the like), and laboratory animal (for example, rat, mouse, guinea pig and the like) in need of prevention or treatment of mucositis or receiving radiotherapy and/or chemotherapy. In an embodiment of the present disclosure, the subject is a human.

The term "treatment" includes prophylactic and/or therapeutic treatment. The prophylactic and/or therapeutic treatment includes all types of treatment recognized in the art and includes, for example, administering the composition or SOD of the present disclosure to a subject. If it is administered prior to clinical manifestation of an unwanted or undesirable condition (for example, disease or other unwanted or undesirable state in the subject), such treatment is prophylactic treatment (for example, it protects the subject against developing the unwanted or undesirable condition). On the other hand, if it is administered after clinical manifestation of an unwanted or undesirable condition, such treatment is therapeutic treatment, such as to diminish, ameliorate, or stabilize the existing unwanted or undesirable condition or side effects thereof.

The meaning of the term "prevention" is well known in the art. When used in the context of a medical condition such as mucositis, it means reducing frequency or delaying onset and symptoms of a medical condition (for example, weight loss, mucosal inflammation or ulcer, diarrhea, and high ROS level in the gastrointestinal tract (for example, small intestine) or blood) in a subject who has received the pharmaceutical composition or SOD of the present disclosure, as compared with a subject who did not receive the same.

The term "administration" refers to providing an active ingredient to a subject to achieve a prophylactic or therapeutic purpose (for example, prevention or treatment of mucositis).

The term "application" means any method of bringing an active ingredient into contact with a biological surface (for example, skin or mucosa) to achieve a prophylactic or therapeutic purpose (for example, prevention or treatment of mucositis), wherein such application is intended to cause absorption of the composition into the skin or mucosa.

### Mucositis

The present disclosure is based, at least in part, on the discovery that administration of a superoxide dismutase (SOD), in particular, oral application or administration thereof, is effective in preventing or treating mucositis. Therefore, according to an aspect of the present disclosure, there is provided a use of an SOD for preventing or treating mucositis.

The term "mucositis" refers to a disease in which inflammation or ulceration occurs in the mucosa of the oral cavity and digestive tract, from the mouth to the anus.

**In** an embodiment, mucositis may occur as a side effect of cancer treatment, including, for example, anticancer treatment (for example, chemotherapy, radiotherapy, or a combination thereof). Conditioning regimens involving chemotherapy and/or radiotherapy for hematopoietic stem cell transplantation (HSCT) and the like may cause mucositis in many patients. In addition, mucositis may result from mucosal damage caused by viral, fungal, or bacterial infection, such as herpes simplex virus (HSV) or candida, and may be induced by a number of diseases or conditions, including neutropenia and autoimmune diseases, such as systemic lupus erythematosus (SLE), Behcet's disease (BD), and the like.

The radiotherapy means a prophylactic or therapeutic method for diseases using radiation or a medical treatment method using radiation, and includes any radiotherapy that causes mucositis, either alone or in combination with chemotherapy. In addition, the chemotherapy means a prophylactic or therapeutic method for diseases using chemical drugs or a medical treatment method using chemical drugs, and includes any chemotherapy that may cause mucositis, either alone or in combination with radiotherapy.

**In** an embodiment, the radiotherapy and chemotherapy may be for prevention or treatment of cancer, wherein the cancer may be selected from the group consisting of, but is not limited to, lung cancer, breast cancer, prostate cancer, colon cancer, bone cancer, pancreatic cancer, skin cancer, head or neck cancer, skin and ocular malignant melanoma, uterine cancer, ovarian cancer, rectal cancer, anal cancer, stomach cancer, testicular cancer, uterine cancer, fallopian tube carcinoma, endometrial carcinoma, cervical carcinoma, vaginal carcinoma, female vulvar carcinoma, Hodgkin's disease, non-Hodgkin's lymphoma, esophageal cancer, small intestine cancer, endocrine system cancer, thyroid cancer, parathyroid cancer, adrenal cancer, soft tissue sarcoma, urethral cancer, penile cancer, chronic and acute leukemia (for example, acute myeloid leukemia, chronic myeloid leukemia, acute lymphoblastic leukemia, or chronic lymphocytic leukemia), childhood solid tumor, lymphocytic lymphoma, bladder cancer, kidney cancer, ureteral cancer, renal pelvis carcinoma, neoplasia in the central nervous system (CNS), primary CNS lymphoma, tumor angiogenesis, spinal cord axis tumor, brainstem glioma, pituitary adenocarcinoma, Kaposi's sarcoma, epidermoid carcinoma, squamous cell carcinoma, T-cell lymphoma, and environmentally induced cancer (for example, asbestos-induced cancer).

The chemotherapy for prevention or treatment of cancer may be chemotherapy using anticancer agents, and mucositis may be induced as a side effect thereof. Using the anticancer agents comprises using the anticancer agents alone or in combination, and may also comprise administering the anticancer agents and other substances (for example, drugs other than anticancer agents, drugs that suppress side effects of anticancer agents, or the like), simultaneously, sequentially, or in reverse order, to induce mucositis.

In an embodiment, the anticancer agent that is capable of causing mucositis may be, but is not limited to, cytotoxic anticancer agents, targeted anticancer agents, immune anticancer agents, or combinations thereof.

The cytotoxic anticancer agents, which are drugs that attack rapidly dividing cells, may include, for example, nucleoside analogs (for example, azacitidine, capecitabine, carmofur, cladribine, clofarabine, cytarabine, decitabine, floxuridine, fludarabine, fluorouracil, gemcitabine, mercaptopurine, nelarabine, pentostatin, tegafur, tioguanine, or the like), antifolates (for example, methotrexate, pemetrexed, raltitrexed, or the like), antimetabolites (for example, hydroxycarbamide or the like), topoisomerase I inhibitors (for example, irinotecan, topotecan, or the like), anthracyclines (for example, daunorubicin, doxorubicin, epirubicin, idarubicin, or the like), podophyllotoxins (for example, etoposide, teniposide, or the like), taxanes (for example, cabazitaxel, docetaxel, paclitaxel, or the like), vinca alkaloids (for example, vinblastine, vincristine, vindesine, vinflunine, vinorelbine, or the like), alkylating agents (for example, bendamustine, busulfan, carmustine, chlorambucil, chlormethine, cyclophosphamide, dacarbazine, fotemustine, ifosfamide, lomustine, melphalan, streptozotocin, temozolomide, or the like), platinum compounds (for example, carboplatin, cisplatin, nedaplatin, oxaliplatin, or the like), or other drugs (for example, altretamine, bleomycin, bortezomib, dactinomycin, estramustine, ixabepilone, mitomycin, procarbazine, or the like), but are not limited thereto.

In addition, the targeted anticancer agents, which are drugs that selectively attack cancer cells by targeting specific portions of the cancer cells which differ from other cells (for example, normal cells), may be, for example, monoclonal antibodies (for example, alemtuzumab, bevacizumab, cetuximab, denosumab, gemtuzumab ozogamicin, ibritumomab tiuxetan, ipilimumab, nivolumab, ofatumumab, panitumumab, pembrolizumab, pertuzumab, rituximab, tositumomab, trastuzumab, or the like), tyrosine kinase inhibitors (for example, afatinib, aflibercept, axitinib, bosutinib, crizotinib, erlotinib, gefitinib, imatinib, lapatinib, nilotinib, pazopanib, ponatinib, regorafenib, ruxolitinib, sorafenib, sunitinib, vandetanib, or the like), mTOR inhibitors (for example, everolimus, temsirolimus, or the like), retinoids (for example, alitretinoin, bexarotene, isotretinoin, tamibarotene, tretinoin, or the like), immunomodulators (for example, lenalidomide, pomalidomide, thalidomide, or the like), histone deacetylase inhibitors (for example, panobinostat, romidepsin, valproate, vorinostat, or the like), or other drugs (for example, anagrelide, arsenictrioxide, asparaginase, BCG vaccine, denileukin diftitox, vemurafenib, or the like), but are not limited thereto.

In addition, the immune anticancer agents, which are drugs that attack cancer cells by utilizing the body's immune system, may be, for example, immune checkpoint inhibitors (for example, atezolizumab, ipilimumab, avelumab, nivolumab, pembrolizumab, durvalumab, or the like), immune cell function enhancers (for example, blinatumomab or the like), or other drugs (for example, alemtuzumab, ofatumumab, elotuzumab, or the like), but are not limited thereto. A single drug may be both a targeted anticancer agent and an immune anticancer agent.

In particular, in a specific embodiment, the anticancer agent that is capable of causing mucositis may be, but is not limited to, 5-fluorouracil (5-FU), cyclophosphamide (CPA), docetaxel, doxorubicin, vincristine, prednisone, etoposide, ifosfamide, methotrexate, paclitaxel, gemcitabine, vinorelbine, leucovorin, irinotecan, oxaliplatin, or a combination thereof.

In an embodiment, the mucositis may be, but is not limited to, oral mucositis (for example, oral ulcer, recurrent oral ulceration, or pharyngeal mucositis), esophageal mucositis, gastrointestinal mucositis (for example, intestinal inflammatory disease or intestinal ulcer disease), rectal mucositis, or a combination thereof.

In an embodiment, the mucositis may cause one or more symptoms such as ulcer or inflammation, pain induction, difficulty eating, diarrhea, weight loss, reduced ROS level, bleeding, reduced epithelial thickness, erythema, and the like. Specifically, the mucositis may cause one or more symptoms selected from the group consisting of ulcer or inflammation, pain induction, difficulty eating, diarrhea, weight loss, reduced ROS level, bleeding, reduced epithelial thickness, and erythema.

In another embodiment, administration of an SOD to a subject in need of prevention, amelioration, or treatment of mucositis may bring about effects such as reduced difficulty eating, ulcer healing, improved re-epithelialization, improved diarrhea symptoms, and improved intestinal villus atrophy in the subject. Specifically, the administration of an SOD may bring about one or more effects selected from reduced difficulty eating, ulcer healing, improved re-epithelialization, improved diarrhea symptoms, and improved intestinal villus atrophy in the subject. In addition, the SOD may bring about an effect of reducing reactive oxygen species (ROS) level in the gastrointestinal tract (for example, small intestine) or blood associated with mucositis.

### Superoxide dismutase (SOD)

According to another aspect of the present disclosure, there is provided an improved superoxide dismutase (SOD) that has an effect of preventing, ameliorating, or treating mucositis.

A superoxide dismutase (SOD) is an enzyme that alternately catalyzes dismutation of superoxide (O₂ ˙ ⁻) radical into either ordinary molecular oxygen (O₂) or hydrogen peroxide (H₂O₂). SODs play a key role in decreasing oxidative stress by removing reactive oxygen species. SODs are widely distributed in prokaryotic and eukaryotic cells and are classified into four classes based on their different types of metal centers (copper/zinc, nickel, manganese, and iron). Manganese-containing SODs (Mn-SODs) are widely present in many bacteria, chloroplasts, mitochondria, and cytosol of eukaryotic cells. The term "SOD" may be used interchangeably with a polypeptide having superoxide dismutase activity.

In an embodiment, the SOD may bind to manganese (Mn-SOD). Specifically, the SOD may be a deamidated Mn-SOD. More specifically, amino acid residues 73 and 136 of the SOD may be substituted with Asp residues, with respect to SEQ ID NO: 2. Even more specifically, the SOD may comprise or consist of the amino acid sequence represented by SEQ ID NO: 4.

The SOD or polypeptide having SOD activity of the present disclosure is interpreted to include amino acid sequences showing substantial identity to the above-mentioned amino acid sequence. The substantial identity means that in a case where aligned amino acid sequences are analyzed using an algorithm commonly used in the art, the sequences show sequence identity of 80% or higher, preferably 90% or higher, more preferably 95% or higher, and most preferably 98% or higher.

In another embodiment, the SOD may be a modified or engineered polypeptide having SOD enzymatic activity, and the polypeptide may comprise one or more mutations, for example, deletion, insertion, or substitution of one or more amino acids, which may or may not affect various aspects (in vivo, in vitro, or ex vivo stability, homogeneity, and/or conformational change). In addition, the polypeptide may further comprise a heterologous substance (for example, a tag known in the art, including HIS tag, HA tag, and myc tag, GFC, and/or Fc domain of an antibody) for purification, detection, or increased stability.

In an embodiment, the SOD may be derived from natural or recombinant microorganisms, and may be an enzyme produced through a process of isolation or purification from various sources.

In an embodiment, the SOD may be derived from natural or recombinant microorganisms. For example, the SOD may be derived from bacteria. Preferably, the SOD may be derived from bacteria that are generally regarded as safe (GRAS) for use in drugs or foods. Specifically, the SOD may be derived from a Bacillus species strain. More specifically, the SOD may be derived from a *Bacillus amyloliquefaciens* strain. For example, the SOD may be derived from the *Bacillus amyloliquefaciens* strain GF423 (KCTC 13222 BP). The GF423 (KCTC 13222 BP) strain was deposited with the Korea Research Institute of Bioscience and Biotechnology on March 6, 2017.

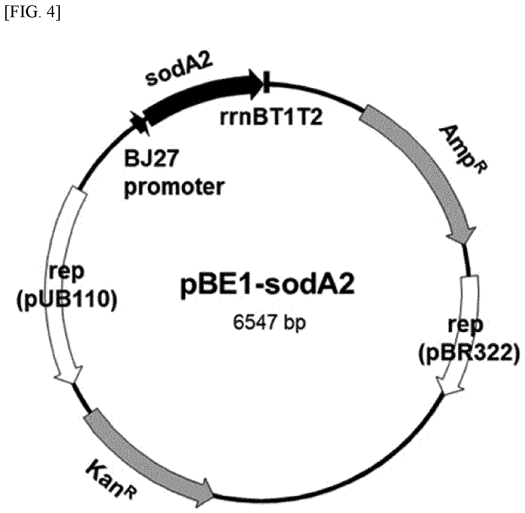

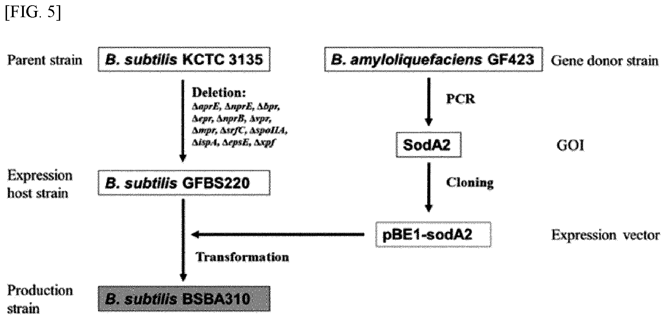

In another embodiment, the SOD may be derived from a recombinant strain comprising the expression vector shown in FIG. 4. In addition, the SOD may be derived from a recombinant strain produced by a method including the method shown in FIG. 5. The recombinant strain may be a Bacillus species strain that comprises a nucleotide sequence encoding a polypeptide that comprises the amino acid sequence of SEQ ID NO: 4. In addition, the recombinant strain may be a Bacillus species strain in which the following genes are deleted: *AprE, NprE, Bpr, Epr, NprB, Vpr, Mpr, IspA, SrfAC, spoIIAc, EpsE,* and *Xpf.* For detailed procedures for preparing the strain, see Example 1.

From the viewpoint that the SOD derived from the above-mentioned strain is an enzyme secreted extracellularly, in a case where an SOD is produced using the strain, it is possible to mass-produce an SOD, which is safe for humans, without going through an expensive purification process (for example, column purification), and this enables efficient production.

In an embodiment, the SOD may be isolated or purified from a variety of sources, including natural or recombinant hosts. For example, the SOD having activity of preventing or treating mucositis may be extracted from a culture supernatant of the *Bacillus amyloliquefaciens* strain GF423. Briefly, the Bacillus *amyloliquefaciens* strain GF423 may be first cultured in various types of media to obtain a culture. For example, the strain is grown at about 25°C to about 42°C for 1 day to 4 days using a complex medium (pH 6.0 to 7.0). Other suitable media for culturing the *Bacillus amyloliquefaciens* strain GF423 include Luria-Bertani (LB) medium, International Streptomyces Project (ISP) medium, nutrient agar (NA) medium, brain heart infusion agar (BHI) medium, sabouraud dextrose agar (SDA) medium, potato dextrose agar (PDA) medium, nutrient broth (NB) medium, and the like. Preferably, LB medium, ISP medium, BHI medium, SDA medium, or NB medium may be used. In addition, the SOD may be obtained from other natural or recombinant hosts using information provided in databases such as PubMed or BRENDA (www.brenda-enzymes.org).

In an embodiment, the SOD may be an isolated or purified enzyme. Here, the isolated or purified SOD, or a biologically active portion thereof is substantially free of cellular material or other contaminating proteins from the cell or tissue source from which it is derived. For example, the purified product may be separated purely from a strain culture through ultrafiltration, ammonium sulfate treatment, column purification, concentration, or the like, or may be a culture concentrate obtained therefrom through ultrafiltration, concentration, or the like. The phrase "substantially free of cellular material" includes preparations of a protein obtained by separating the protein from cellular components of the cell from which the protein is isolated or recombinantly produced. In an embodiment, the phrase "substantially free of cellular material" includes preparations of a protein in which unwanted proteins are present in an amount of less than about 30%, preferably less than about 20%, more preferably less than about 10%, and most preferably less than about 5% by dry weight.

In another embodiment, the SOD may be preferably purified by the following purification method, but the method is not limited thereto. The culture obtained by culturing the *Bacillus amyloliquefaciens* strain GF423 is centrifuged to collect a culture supernatant. The supernatant fraction is pretreated by solid-phase extraction, and then isolated and purified by chromatography. Here, various modes of chromatography may be used to purify the SOD. Preferably, hydrophobic interaction chromatography is used.

In an embodiment, the SOD may be included in a form of a strain lysate, a strain culture, a strain culture concentrate, or a strain culture extract, or a dried form thereof. Here, the "strain lysate" may be obtained by culturing a strain and disrupting it mechanically or chemically, and may include any material obtained therefrom after additional processes such as extraction, dilution, concentration, purification, and the like. The "strain culture" may refer to a culture itself obtained by culturing a strain or a supernatant thereof. The "strain culture concentrate" refers to a product that is separated purely from a strain culture through ultrafiltration, ammonium sulfate treatment, column purification, concentration, or the like, or a culture concentrate obtained therefrom through ultrafiltration, concentration, or the like. The "strain culture extract" refers to a product that is extracted from the culture or a concentrate thereof, and may include an extract, a diluted or concentrated solution of the extract, a dried product obtained by drying the extract, or a crude or purified product thereof, or a fraction obtained by fractionating the same. The dried form may include a lyophilized form.

According to an embodiment of the present disclosure, it has been identified that in a case of being administered to a subject in need of prevention or treatment of mucositis, the SOD exhibits one or more effects such as reduced difficulty eating in the stomach, ulcer healing, improved re-epithelialization, improved diarrhea symptoms, and improved intestinal villus atrophy. According to another embodiment, in a case of being applied in the oral cavity or administered orally to a subject in need of prevention or treatment of mucositis, the SOD exhibits an effect of reducing an ROS (Reactive Oxygen Species) level in the gastrointestinal tract (for example, small intestine) or blood.

### Dosage

The present disclosure has been completed, at least in part, by confirming that the SOD of the present disclosure is effective in prevention or treatment of mucositis even in a case of being applied in the oral cavity or administered orally at low doses.

The SOD of the present disclosure may be administered at a dose of about 50 units/kg/day to about 2,500 units/kg/day, preferably about 125 units/kg/day to about 1,000 units/kg/day, more preferably about 125 units/kg/day to about 500 units/kg/day or about 250 units/kg/day to about 1,000 units/kg/day, and even more preferably about 250 units/kg/day, about 500 units/kg/day, or about 1,000 units/kg/day, based on a subject's weight. In particular, when the SOD of the present disclosure is applied in oral cavity, it can effectively exhibit an effect of preventing or treating mucositis even in a case where its dosage is reduced to half of the oral administration dosage based on a subject's weight. For example, when the SOD is applied in the oral cavity, it may be applied at a dose of about 25 units/kg/day to about 1,250 units/kg/day, preferably about 125 units/kg/day to about 500 units/kg/day, and more preferably about 250 units/kg/day, about 500 units/kg/day, or about 1,000 units/kg/day.

### Pharmaceutical composition

According to yet another aspect of the present disclosure, there is provided a pharmaceutical composition for preventing or treating mucositis, comprising a superoxide dismutase (SOD) as an active ingredient.

**In** an embodiment, the SOD according to the present disclosure may be combined with a pharmaceutically acceptable carrier, excipient and/or diluent to form a pharmaceutical composition for prevention or treatment of mucositis. The pharmaceutical composition of the present disclosure may be used interchangeably with the term veterinary composition when applied to an animal other than a human.

The pharmaceutical or veterinary composition of the present disclosure may further comprise one or more selected from the group consisting of pharmaceutically acceptable carriers, excipients, and diluents. The pharmaceutically acceptable carrier, excipient, and/or diluent may be those commonly used in the art. The carrier, excipient, or diluent may include, for example, lactose, dextrose, sucrose, sorbitol, mannitol, xylitol, erythritol, maltitol, starch, gum acacia, alginate, gelatin, calcium phosphate, calcium silicate, cellulose, methyl cellulose, hydroxypropyl methyl cellulose, microcrystalline cellulose, polyvinyl pyrrolidone, water, methyl hydroxybenzoate, propyl hydroxybenzoate, talc, magnesium stearate, silicon dioxide, and mineral oil, but are not limited thereto.

In a case of performing preparation, the preparation may be achieved by using an additive such as a filler, an extender, a binder, a wetting agent, a disintegrant, or a surfactant. The additive for preparation may be appropriately selected from those commonly used in the pharmaceutical field.

In addition, the pharmaceutical or veterinary composition of the present disclosure may be formulated into a desired form depending on the method of use, and in particular, the formulation may be achieved by employing a method known in the art to provide rapid, sustained, or delayed release of the active ingredient after being administered to a mammal. Specific examples of such a formulation include tablet, pill, powder, granule, syrup, solution, capsule, suspension, emulsion, injection solution, plaster, lotion, liniment, lemonade, aerosol, extract, elixir, ointment, fluid extract, needle, cream, soft or hard gelatin capsule, patch, and the like.

Furthermore, the pharmaceutical or veterinary composition of the present disclosure may preferably be formulated using an appropriate method known in the art or a method disclosed in Remington's Pharmaceutical Science (latest edition), Mack Publishing Company, Easton PA.

In an embodiment, the composition may be administered by any conventional route capable of reaching the target *in vivo.* For example, it may be administered by oral application, oral administration, parenteral administration routes including intradermal, intravenous, intramuscular, intraarterial, intramedullary, intrathecal, intraventricular, intrapulmonary, transdermal, subcutaneous, intraperitoneal, intranasal, intragastric, topical, sublingual, vaginal, or rectal routes, but the route is not limited thereto. In a case of being orally administered, the SOD may be coated with shellac for protection from gastric acid, but the coating agent is not limited thereto. Examples of the coating agent suitable for use in the present disclosure include shellac, ethyl cellulose, hydroxypropyl methylcellulose, hydroxypropyl methylcellulose phthalate, zein, Eudragit, and combinations thereof. In a case where the SOD is coated, the SOD may be coated in solution. Specifically, a purified solution and a shellaccontaining solution are mixed and then lyophilized. This lyophilized sample may be made into powder and stored at about 4°C until use. Solid preparations for oral administration include tablets, pills, powders, granules, capsules and the like, and these solid preparations may be prepared by mixing a composition with at least one excipient, such as starch, calcium carbonate, sucrose, lactose, gelatin and the like. In addition to simple excipients, a lubricant such as magnesium stearate and talc may also be used. Examples of liquid preparations for oral administration include suspensions, oral solutions, emulsions, and syrups. In addition to commonly used simple diluents such as water and liquid paraffin, various types of excipients, such as a wetting agent, a sweetener, a fragrance, and a preservative, may be used.

The composition according to the present disclosure may be applied in the form of a topical agent or administered in the form of an injection. In addition, the composition according to the present disclosure may be administered by any device that allows the active ingredient to be delivered to target cells.

In addition, the pharmaceutical or veterinary composition of the present disclosure is administered in a pharmaceutically or veterinary effective amount. The term "pharmaceutically effective amount" or "veterinary effective amount" refers to an amount sufficient to treat a disease at a reasonable benefit/risk ratio applicable to any medical or veterinary treatment. A level of the effective amount may be determined depending on factors including the patient's or animal's body weight, gender, age, health status, severity of disease, activity of the drug, sensitivity to the drug, administration time, administration route and excretion rate, duration of treatment, and drugs used concurrently therewith, and other factors well known in the medical field. Preferably, the pharmaceutical or veterinary composition of the present disclosure may comprise an SOD in an amount of about 2 to about 1,500 U/mg, specifically about 5 to about 1,000 U/mg, more specifically about 10 to about 800 U/mg, and even more specifically about 100 to about 500 U/mg, based on the total weight of the composition.

The pharmaceutical or veterinary composition of the present disclosure may be administered as an individual therapeutic agent or in combination with other therapeutic agents, in which the administration may be carried out in a sequential or simultaneous manner. In addition, the pharmaceutical composition may be administered once or multiple times as needed. Taking all of the above factors into consideration, it is important to administer a minimum amount that allows the maximum effect to be obtained with no adverse effects, and such an amount may be easily determined by those skilled in the art.

In an embodiment, the composition of the present disclosure may be administered simultaneously with, sequentially, or in reverse order to radiotherapy and/or chemotherapy. Specifically, the composition of the present disclosure may be administered before, after, or both before and after performing radiotherapy and/or chemotherapy.

### Therapeutic or prophylactic method

According to still yet another aspect of the present disclosure, there is provided a method for treating or preventing mucositis, comprising administering, to a subject having or at risk of mucositis, the SOD or composition according to the present disclosure.

The term "administration" includes any of a variety of administrations that accomplish a desired action and enable prevention or treatment. With respect to the route of administration, see the details described above. For example, the method may comprise administering a therapeutically effective amount of the SOD or composition of the present disclosure to one or more regions of the digestive tract of a subject having or at risk of developing mucositis. Furthermore, the method may comprise applying a therapeutically effective amount of the SOD or composition of the present disclosure to the oral cavity of a subject having or at risk of developing oral mucositis.

An effective amount or effective non-toxic amount of the SOD according to the present disclosure may be determined by conventional experimentation. For example, a therapeutically active amount of the SOD of the present disclosure may vary depending on factors such as disease stage, disease severity, subject's age, gender, medical complications, and body weight. Dosage and dosing regimen of the SOD of the present disclosure may be adjusted to provide an optimal therapeutic response. For example, several divided doses may be administered daily, weekly, every two weeks, every three weeks, every four weeks and the like, and/or the dose may be proportionally reduced or increased depending on exigencies of therapeutic situation.

The method may comprise administering one or more additional agents for treating or preventing mucositis. The additional agents include, but are not limited to, any agent that can improve or ameliorate mucositis, such as compounds, gene therapy agents, proteins (including antibodies), and the like. The one or more additional agents may be administered simultaneously with, sequentially, or in reverse order to the SOD or pharmaceutical composition. In addition, the individual agents may be administered to a subject by the same or different routes.

Meanwhile, in an embodiment, the method may comprise administering the SOD or composition of the present disclosure to a subject before, after, or both before and after receiving radiotherapy.

In another embodiment, the method may comprise administering the SOD or composition of the present disclosure to a subject before, after, or both before and after receiving chemotherapy.

In yet another embodiment, the method may comprise administering the SOD or composition of the present disclosure to a subject who is undergoing anticancer treatment.

Here, the SOD or composition of the present disclosure may be administered in combination with chemotherapy (for example, administration of the anticancer agent) and/or radiotherapy, regardless of the order and frequency thereof.

### Food composition

According to still yet another aspect of the present disclosure, there is provided a food composition for preventing or ameliorating mucositis, comprising the SOD according to the present disclosure as an active ingredient. Such a food composition includes a medical or nutraceutical food composition.

The term "medical food" or "nutraceutical food" refers to a food manufactured from raw materials or ingredients that are likely to have beneficial functions in the human body, the food maintaining normal function or activating physiological functions of the human body to maintain or improve health. This food is defined by the Ministry of Food and Drug Safety, but is not limited thereto. The food does not exclude any common health food from its meaning.

In addition, the food includes, but is not limited to, various foods, food additives, beverages (for example, functional drinks, natural fruit juices, and vegetable drinks), gum, tea, vitamin complexes, health functional foods, other functional foods, and the like.

The food may be manufactured by conventional methods known in the art. For example, the medical food, nutraceutical food, or health functional food may be formulated into one selected from the group consisting of tablet, pill, powder, granule, capsule, and liquid formulation by further comprising at least one of a carrier, a diluent, an excipient, and an additive, in addition to the SOD, for the purpose of preventing or ameliorating mucositis. Specific examples of the carrier, excipient, diluent, and additive are well known in the art, and those skilled in the art are capable of combining appropriate ingredients depending on the formulation to manufacture the food.

An amount of the superoxide dismutase according to the present disclosure as an active ingredient in the above-described formulation may be adjusted appropriately depending on the form and purpose of use, the patient's condition, type and severity of symptoms, and the like. The amount may be, but is not limited to, 0.001 to 99.9% by weight, and preferably 0.01 to 50% by weight, based on the weight of solid content.

A dose of the food of the present disclosure may vary depending on a patient's age, body weight, gender, dosage form, health status, and severity of disease, and the administration may be done once a day or in divided doses several times a day at regular time intervals depending on the judgment of a doctor or pharmacist. For example, the daily dosage may be 10 to 1,000 mg/kg based on the active ingredient content. The dosage is an example of an average case and may increase or decrease depending on individual differences. In a case where the daily dosage of the health functional food of the present disclosure is less than the above-mentioned dosage, it is not possible to obtain significant effects. In a case where the daily dosage exceeds the above-mentioned dosage, it is not only uneconomical but also undesirable side effects may occur because such a dosage is outside the usual dosage range.

### Feed composition

According to still yet another aspect of the present disclosure, there is provided a feed composition for preventing or ameliorating mucositis, comprising the SOD according to the present disclosure as an active ingredient. In the present disclosure, the food composition may include a feed composition. This feed composition of the present disclosure may be prepared in any formulation commonly used in the art. For example, the feed composition of the present disclosure may further comprise an auxiliary ingredient such as amino acids, inorganic salts, vitamins, antibiotics, antibacterial substances, antioxidants, antifungal enzymes, and other microbial agents in the form of live bacteria; grain such as ground or broken wheat, oats, barley, corn, and rice; vegetable protein feed such as those comprising rapeseed, soybean, and sunflower as main ingredients; animal protein feed such as blood meal, meat meal, bone meal, and fish meal; a dry ingredient consisting of sugar and a dairy product such as various powdered milk and whey powder; lipid such as animal fat and vegetable fat, optionally liquefied by heating; and an additive such as nutritional supplements, digestion and absorption enhancers, growth promoters, and disease prevention agents.

The feed composition of the present disclosure may be in a form of a powder or liquid preparation, and may comprise an excipient that is added to feed (calcium carbonate, wheat shorts, zeolite, corn meal, rice bran, or the like).

### Kit

According to still yet another aspect of the present disclosure, there is provided a kit for preventing, ameliorating, or treating mucositis, comprising the SOD or composition according to the present disclosure.

In an embodiment, the kit may further comprise an instruction manual for instructing use of the SOD or composition of the present disclosure. The instruction manual may comprise instructions for taking or administering the active ingredient. For example, the instruction manual may comprise instructions for taking or administering the active ingredient in combination with radiotherapy and/or chemotherapy, or in combination with an anticancer agent. Specifically, the instruction manual may comprise instructions such as timing, order, frequency, dosage, and the like for the active ingredient in combination with radiotherapy and/or chemotherapy, or in combination with an anticancer agent.

Hereinafter, the present disclosure will be described in more detail by way of the following examples. The following examples are presented to help understand the present disclosure. These examples are not intended to limit a scope of the present disclosure in any way and should not be construed as limiting the present disclosure.

### Examples

### Example 1. Preparation of recombinant strain expressing superoxide dismutase (SodA2)

### Example 1.1. Preparation of expression host GFBS220

The expression host GFBS220 was prepared using *Bacillus subtilis* KCTC 3135. *B. subtilis* KCTC 3135 was obtained from the Korea Research Institute of Bioscience and Biotechnology, Biological Resources Center (KCTC). To facilitate post-processing, the genes shown in Table 1 below were removed from the genome of *B. subtilis* KCTC 3135.

**[Table 1]**

| Gene name | Protein name | Function |
|---|---|---|
| *AprE* | Serine alkaline protease (subtilisin E) | Extracellular protease |
| *NprE* | Extracellular neutral metalloprotease | Extracellular protease |
| *Bpr* | Bacillopeptidase F | Extracellular protease |
| *Epr* | Extracellular serine protease | Extracellular protease |
| *NprB* | Extracellular neutral protease B | Extracellular protease |
| *Vpr* | Extracellular serine protease | Extracellular protease |
| *Mpr* | Extracellular metalloprotease | Extracellular protease |
| *IspA* | Intracellular serine protease | Intracellular protease |
| *SrfAC* | Surfactin synthase | Surfactin synthesis |
| *spoIIAC* | RNA polymerase sporulation-specific sigma factor (sigma-F) | Sporulation |
| *EpsE* | Putative glycosyltransferase | Extracellular polysaccharide |
| *Xpf* | RNA polymerase sigma factor | Transcription of PBSX |
| | | prophage gene |

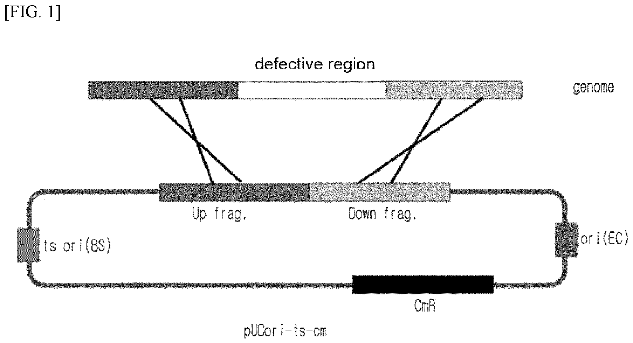

Removal of the genes was performed using double crossover recombination on the genome (FIG. 1). Specifically, DNA segments (up frag. and down frag.), which have homology to the DNA base sequences flanking the gene to be manipulated, were cloned into the vector pUCori-ts-cm that has a temperature-sensitive replication origin. PCR conditions and primers used for cloning are shown in Tables 2 and 3, respectively.

**[Table 2]**

| **PCR steps** | **Temperature** | **Time** |
|---|---|---|
| First denaturation | 95°C | 30 s |
| 30 cycles | 95°C | 30 s |
| | 50°C | 30 s |
| | 72°C | 1 min/kb |
| Final elongation | 72°C | 10 min |
| DNA polymerase used in PCR is *Taq* DNA polymerase (NEB, USA). | | |

**[Table 3]**

| Target gene | Homologous pair | | Primer sequence (5'→3') | SEQ ID NO |
|---|---|---|---|---|
| AprE | fragment 1 | F | ctacggggtctgacgcagatatcgtaaccgaagaacg | 5 |
| | | R | tgttgcagacatgttgctgaacgccatc | 6 |
| | fragment 2 | F | caacatgtctgcaacatatcttggaaac | 7 |
| | | R | gataagctgtcaaaccagagattggataggctatcaag | 8 |
| NprE | fragment 1 | F | ctacggggtctgacgcagggcattactgcaccataatc | 9 |
| | | R | gaggtacgccttcagcagcctgaacacc | 10 |
| | fragment 2 | F | gctgaaggcgtacctcacgccttcttcc | 11 |
| | | R | gataagctgtcaaaccagttgtcagatttgcatccttc | 12 |
| Bpr | fragment 1 | F | ctacggggtctgacgcagagcaatttccagcccgcttc | 13 |
| | | R | gtatgatgaggcatgaacagcaatcccg | 14 |
| | fragment 2 | F | tcatgcctcatcatactcaacaagggtg | 15 |
| | | R | gataagctgtcaaaccagttcccagccggatgttcaac | 16 |
| Epr | fragment 1 | F | ctacggggtctgacgcaggctttctgccagccgatagg | 17 |
| | | R | tgtgtcaaagccgttatgcttggctccg | 18 |
| | fragment 2 | F | taacggctttgacacagcacaaactgcc | 19 |
| | | R | gataagctgtcaaaccaggtctcccatacatgaacgac | 20 |
| NprB | fragment 1 | F | ctacggggtctgacgcagagtaatcttgtaggaggctg | 21 |
| | | R | tgtgactgtcatattcaccgtcgtgtgg | 22 |
| | fragment 2 | F | tgaatatgacagtcacacagtattccgcc | 23 |
| | | R | gataagctgtcaaaccagattaggatacggtctggctg | 24 |
| Vpr | fragment 1 | F | ctacggggtctgacgcagatgtacgctgagccgaatag | 25 |
| | | R | ggaatcacatttgcggagatacggcgtc | 26 |
| | fragment 2 | F | ccgcaaatgtgattccggcacatcaaac | 27 |
| | | R | gataagctgtcaaaccagaccgttttccgaatctgacc | 28 |
| Mpr | fragment 1 | F | ctacggggtctgacgcagcgttatcccgaatgcccgtg | 29 |
| | | R | ggctttgttcctttagtgtcaaccaccc | 30 |
| | fragment 2 | F | ctaaaggaacaaagccgattcgctccgc | 31 |
| | | R | gataagctgtcaaaccagaaattgactgctccacgctg | 32 |
| SrfAC | fragment 1 | F | ctacggggtctgacgcaggatatgtattacctatcgcc | 33 |
| | | R | gccccttcatccagttcactgcttccttg | 34 |
| | fragment 2 | F | ggaagcagtgaactggatgaaggggcttcgctg | 35 |
| | | R | gataagctgtcaaaccagaacgcttcgacatcactttc | 36 |
| spoIIA | fragment 1 | F | ctacggggtctgacgcagagcggacgatgggagactgg | 37 |
| | | R | gccacctcatgcagccgatctggaagag | 38 |
| | fragment 2 | F | ggctgcatgaggtggctgagcggctcgg | 39 |
| | | R | gataagctgtcaaaccagctcattgttttggtgagctg | 40 |
| IspA | fragment 1 | F | ctacggggtctgacgcagtgctgcttggcattcatttc | 41 |
| | | R | gccattgaagcaatgcctccgtttgaatc | 42 |
| | fragment 2 | F | acggaggcattgcttcaatggctgcccctcatg | 43 |
| | | R | gataagctgtcaaaccagtcaatgctctcgtcatattc | 44 |
| EpsE | fragment 1 | F | ctacggggtctgacgcagcaagcaaatgggctgggagg | 45 |
| | | R | gacaagccatgctttctgccaaagtgcg | 46 |
| | fragment 2 | F | gaaagcatggcttgtcaagcatgaatag | 47 |
| | | R | gataagctgtcaaaccagtgttgtatacattcagcagc | 48 |
| Xpf | fragment 1 | F | gatcctctacggttcatccatgtccgaac | 49 |
| | | R | tctatgatcctcctcatttttg | 50 |
| | fragment 2 | F | gaggaggatcatagaaagcttgtcatacgtttgcc | 51 |
| | | R | gagttttcgttcggatcctccgctttttgtttg | 52 |

The thus produced vectors were introduced into *B. subtilis* cells, and then transformants (single crossover homologous recombination) were selected using medium containing chloramphenicol at 37°C (non-replication temperature). The selected transformants were cultured (second crossover) at 30°C (replication-permissive temperature) in LB medium without antibiotics, and then cultured at 39°C in LB agar medium without antibiotics. Colonies susceptible to chloramphenicol were selected from the resulting colonies (plasmid curing). The selected colonies were subjected to PCR to obtain double crossover recombinants, and second pure separation was performed to select the final recombinant, which was named GFBS220.

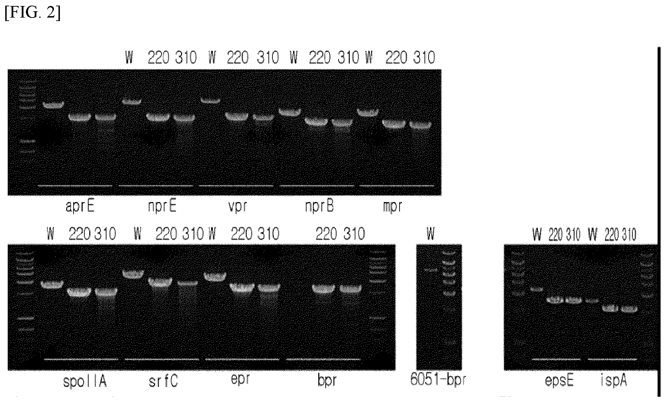

The defective gene regions of GFBS220 were amplified with the primers shown in Table 4 and PCR, and then identified using agarose gel electrophoresis. The results obtained by identifying the defective genes using PCR in the expression host GFBS220 are shown in FIG. 2.

**[Table 4]**

| Gene region (locus) | PCR primer (SEQ ID NO) | Size of PCR product of *B. subtilis* KCTC 3135 | Size of PCR product of GFBSL210 |
|---|---|---|---|
| *AprE* | F caaggcttgaaataacgttg (SEQ ID NO: 53) | 3115bp | 2116bp |
| | R ttcgctgattacaacattgg (SEQ ID NO: 54) | | |
| *NprE* | F ggaacagatgatagctcatc (SEQ ID NO: 55) | 3487bp | 2145bp |
| | R acactccgagaaggctgaag (SEQ ID NO: 56) | | |
| *Vpr* | F ctgcggcctgcacagccgcc (SEQ ID NO: 57) | 3733bp | 2293bp |
| | R cgctgaatgacggtggtaag (SEQ ID NO: 58) | | |
| *NprB* | F agtaatcttgtaggaggctg (SEQ ID NO: 59) | 2666bp | 2153bp |
| | R cgccaatgaagtgaaggagg (SEQ ID NO: 60) | | |
| *Mpr* | F ggttgaggcgattgcgacgg (SEQ ID NO: 61) | 2734bp | 2076bp |
| | R ccattctgcaaaatcagctc (SEQ ID NO: 62) | | |
| *spoIIAC* | F aagatatgaagaaagccggg (SEQ ID NO: 63) | 2628bp | 2102bp |
| | R acagccgcttcataagcctg (SEQ ID NO: 64) | | |
| *ThrC* | F tcaagctgtcatgtacggag (SEQ ID NO: 65) | 376bp | 5455bp |
| | R tccgatacgaatggctgtcg (SEQ ID NO: 66) | | |

### Example 1.2. Construction of expression vector pBE1-sodA2

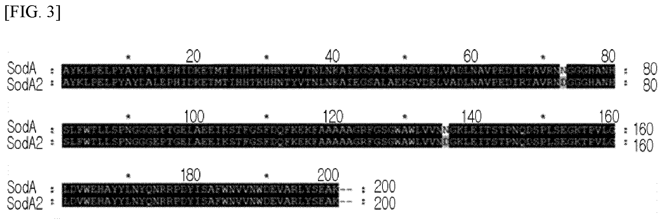

An Mn-SOD was identified in the culture supernatant of *Bacillus amyloliquefaciens* strain GF423 through N-terminal sequencing (Kang et al., 2018). The GF423 strain was deposited with the Korea Research Institute of Bioscience and Biotechnology on March 6, 2017 (under accession number of KCTC 13222 BP). The gene of the Mn-SOD was named *sodA* according to the nomenclature for bacterial SOD. For the amino acid sequence thereof, see FIG. 3 and SEQ ID NO: 2 (for the nucleotide sequence thereof, see SEQ ID NO: 1). LC-UV-MS/MS peptide mapping of the enzyme preparation produced 100% amino acid sequence coverage; however, deamidation was observed at Asn73 and Asn136 residues at 73% and 17% abundance, respectively. Therefore, to improve homogeneity of the purified enzyme, both Asn residues were replaced with Asp residues. For the amino acid sequence of such variant sodA, named SodA2, see FIG. 3 and SEQ ID NO: 4 (for the nucleotide sequence thereof, see SEQ ID NO: 3).

The *sodA2* gene was amplified with duplicate elongation PCR using four primers (Table 5) and the genome of *Bacillus amyloliquefaciens* GF423 as a template. Nucleotides for replacement of Asn with Asp are underlined.

**[Table 5]**

| Primer | | Sequence (5'→3') | SEQ ID NO |
|---|---|---|---|
| 1 | SOD F | GGAGGAATTACAATGGCTTACAA | 45 |
| 2 | D74 R | TGCATGTCCGCCGCCATCGTTGCGGAC | 46 |
| 3 | D74 F | CAGTCCGCAACGATGGCGGCGGACATG | 47 |
| 4 | D137 R | TTCAAGTTTGCCGTCGTTTACAACGAGC | 48 |
| 5 | D137 F | CTCGTTGTAAACGACGGCAAACTTG | 49 |
| 6 | SOD R | CAAAACAGAAGCTTCATTATTTTGCT | 50 |

The amplified DNA fragment containing the *sodA2* gene and the plasmid pBE1 linearized by treatment with *NdeI* and *HindIII* were assembled *in vitro* by the SLIC method (Jeong et al. 2012), and then the resulting construct was transformed into *E. coli* C2984H. Correct clones were screened by restriction enzyme mapping and identified by nucleotide sequencing. The resulting expression vector pBE1-sodA2 is shown in FIG. 4.

### Example 1.3. Preparation of production strain BSBA310

The expression vector pBE1-sodA2 was transformed into *B. subtilis* GFBS220. Strains were selected from the transformants, and pure clones were established by two single colony isolation procedures in LB2 medium. The selected strain was named BSBA310 and stored at -80°C using the glycerol stock method. The results obtained by identifying the defective genes using PCR in this production strain BSBA310 are shown in FIG. 2. In addition, the overall procedure for preparing the production strain BSBA310 is summarized in FIG. 5.

### Example 2. Isolation and purification of superoxide dismutase (SodA2) from production strain BSBA310

### Example 2.1. Culture of production strain BSBA310

For culture of the production strain BSBA310 obtained in Example 1, the single colony formed on LB agar medium (LB (Luria-Bertani) agar; 10 g/L of tryptophan, 5 g/L of yeast extract, 10 g/L of NaCl, 15 g/L of agar) was inoculated into 30 ml of LB medium and cultured at 37°C for 12 hours. This seed culture was again inoculated into 3 L of LB medium containing 1 mM manganese sulfate (MnSO₄), and cultured at 37°C for 20 hours.

### Example 2.2. Isolation and purification of SodA2

The cell culture obtained in Example 2.1 was centrifuged at 4 °C and 3,578 x g for 20 minutes to collect the supernatant, which was then concentrated 10-fold using ultrafiltration (hereinafter referred to as UF, MWCO 10,000). 390 g of ammonium sulfate was added thereto per liter of the concentrated cell culture, the mixture was stirred for 20 minutes, and centrifugation was performed to take the supernatant. The taken supernatant was purified using a phenyl Sepharose HP column. The purification process was as follows. The column was equilibrated using 50 mM potassium phosphate pH 7.0 with a concentration of 2 M ammonium sulfate. Then, the supernatant obtained by adding ammonium sulfate was passed through the column, and SodA2 attached to the column was recovered using 50 mM potassium phosphate pH 7.0 containing 1.6 M ammonium sulfate. The solution purified through the column was collected and concentrated by ultrafiltration while removing highly concentrated salts formed during the purification process. The concentrate was filtered through a sterilization filter and then lyophilized. Activity of SodA2 was analyzed using the SOD assay kit (Cayman Chemical, Michigan, USA). One unit of SOD activity is defined as an amount of the enzyme that inhibits superoxide radicals by 50%. Activity of the dried SodA2 enzyme ranged from 1,000 to 1,500 U/mg.

### Example 3. Preparation of substance to be administered

For the intragastric administration group that receives oral administration, shellac (EXCELACS Co., Ltd., Bangkok) was dissolved in ethanol to a concentration of 3% and sterilized using a 0.2 µm sterile filter. The shellac dissolved in ethanol was diluted with PBS. Lyophilized SodA2 was dissolved at 20 mg/mL, and the shellac solution and SOD solution were mixed in a 1:1 ratio, and then lyophilized. The substance used in the animal test was prepared by mixing the lyophilized shellac-coated SodA2 with dextrin in a ratio of 1:9 to 12 (shellac-coated SodA2: dextrin). The prepared SOD was adjusted to have activity of 90 to 110 U/mg. For the intragastric administration group, the substance was diluted to a final concentration of 10 U/mg and used.

For the oral application group, SodA2 was suspended in 2% methylcellulose (Sigma) gel and used. For the oral application group, the SOD activity was adjusted to 100 U/mg.

### Example 4. Production of mucositis model and administration of SodA2

To produce a mucositis model, 6-week-old Sprague Dawley rats were purchased (from Orient Bio) and acclimatized for 1 week, and then randomly divided into 8 groups. The 8 groups are as follows: G1 (control group), G2 (disease group), oral application groups (G3, G4, and G5), and intragastric administration groups that receive oral administration (G6, G7, and G8). For the test groups, dosage concentrations, and number of animals of the mucositis model, see Table 6. Mucositis was induced by intraperitoneally administering 5-fluorouracil (purchased from Sigma, 100 mg/kg) twice on days 1 and 3, performing anesthesia with ketamine (purchased from Yuhan Corporation; 40 mg/kg) on day 5, and creating a wound in the left oral mucosa with an 18-gauge needle to simulate chronic irritation caused by masticatory movements in patients undergoing anticancer treatment so that mechanical ulcerative lesions are induced. Sham operation was performed for the control group G1.

**[Table 6]**

| Test group | | Dosage concentration | | Number of animals |
|---|---|---|---|---|
| | | mg/kg/day | SOD U/kg/day | |
| G1 | Control group | Vehicle administration | | 8 |
| G2 | Disease group | Vehicle administration | | 8 |
| G3 | Oral application group (low concentration) | 1.25 | 125 | 8 |
| G4 | Oral application group (medium concentration) | 2.5 | 250 | 8 |
| G5 | Oral application group (high concentration) | 5 | 500 | 8 |
| G6 | Intragastric administration group (low concentration) | 25 | 250 | 8 |
| G7 | Intragastric administration group (medium concentration) | 50 | 500 | 8 |
| G8 | Intragastric administration group (high concentration) | 100 | 1,000 | 8 |

For the oral application group, the test substance was prepared by being suspended in 2% methylcellulose gel as a vehicle, and then 100 µl was applied using a micropipette so that the entire drug can be applied to the oral mucosa. For the intragastric administration group, the test substance was administered orally (by oral gavage) at a dose of 10 mL/kg using PBS as a vehicle. The administration was conducted over a period of 10 days after completion of mechanical surgery to induce oral mucositis until ulcer healing in the normal group, and the administration was performed once a day at around 10:00 AM. The vehicle was administered only to G1 (control group) and G2 (disease group).

### Example 5. Measurement of body weight and food intake

To evaluate energy absorption impairment caused by oral and gastrointestinal mucositis, body weight and food intake were measured at intervals of 2 to 3 days. Measurement of food intake was performed by weighing the food before feeding a cage the previous day, and then measuring the remaining food the next day to calculate the intake, wherein the average food intake was calculated by dividing the total food intake by the number of animals in the cage.

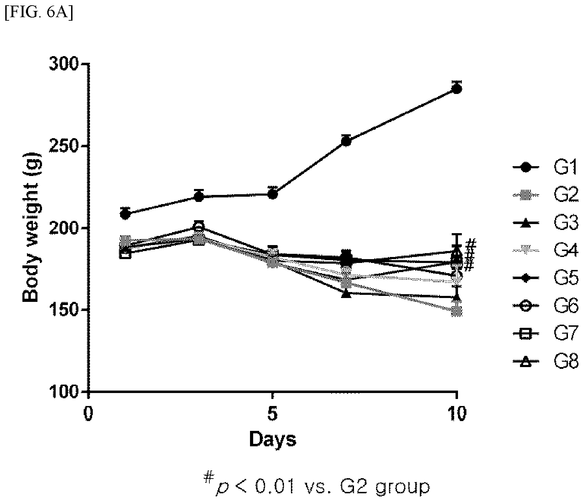

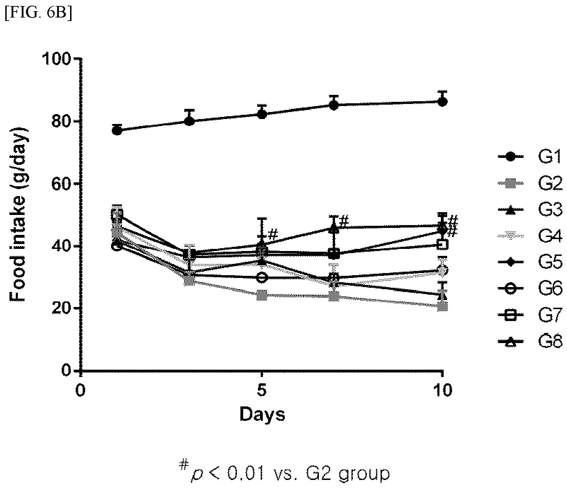

For the normal group G1, the body weight and food intake increased steadily for 10 days. On the contrary, for G2 to G8 with induced mucositis, the food intake reduced significantly compared to G1, and the body weight increased slightly at the beginning and then tended to continuously decrease thereafter. On the other hand, for the drug administration groups (G3 to G8), an increase in body weight and food intake was observed compared to G2. In particular, the body weight increased statistically significantly in G5, G7, and G8 which are the groups administered with SodA2 at medium and high concentrations, and the food intake increased statistically significantly in G5 and G8 which are the groups administered with SodA2 at high concentrations. The graphs for body weight and food intake of the test groups are shown in FIGS. 6A and 6B.

### Example 6. Scoring and histological evaluation of oral mucositis

### Scoring of oral mucositis

The ulcer site was observed at intervals of 3 to 4 days immediately after induction of mechanical ulceration, and scoring of oral mucositis was performed according to the criteria in Table 7 below. For the scoring criteria, see Araujo et al. PLoS One 2015, 10: e0116799.

**[Table 7]**

| Score | Criteria |
|---|---|
| 0 | Healthy mucosa and no evidence of erosion or vasodilation |
| 1 | Presence of erythema and no evidence of mucosal erosion |
| 2 | Severe erythema, vasodilation, and surface erosion |
| 3 | Ulcer present on at least one side of mucosa, 25% or less of area affected, severe erythema, and vasodilation |
| 4 | Ulcer present on approximately 50% of buccal mucosal area |
| 5 | Completely ulcerated buccal mucosa that precludes possibility of tissue exposure |

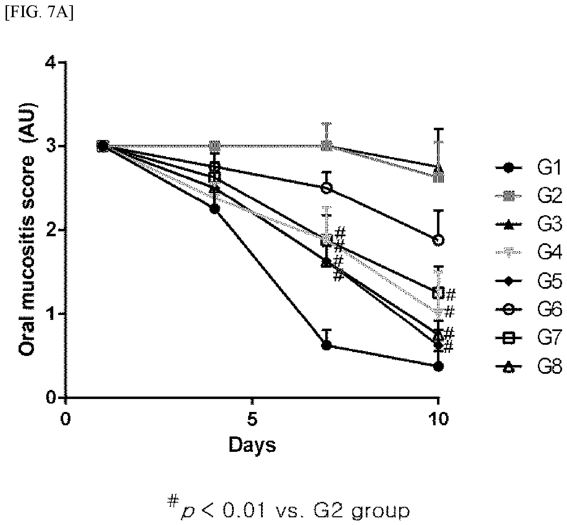

Regarding oral mucositis, the ulcer site was analyzed for the degree of re-epithelialization for 10 days after mechanical ulceration. In the normal group G1, the ulcer site recovered quickly and re-epithelialization was completed by day 10, with only minor traces of the wound observable. On the other hand, for the G2 group, differentiation of epithelial cells in the ulcer site was suppressed by 5-FU, and thus re-epithelialization hardly occurred, wherein some individuals showed a tendency for the ulcer site to enlarge due to continuous masticatory irritation. In the drug administration group, it was confirmed that re-epithelialization was promoted in a concentration-dependent manner, leading to healing of the ulcer site (FIG. 7A).

### Histological evaluation of oral mucositis

Tissues containing the oral mucosa were collected, fixed in 10% formalin, and then subjected to standard histological processing to prepare hematoxylin and eosin (H&E)-stained slide glasses. Subsequently, the lesion site caused by mucositis was examined using a microscope to evaluate the degree of re-epithelialization of the mucositis area and analyze the thickness of the oral mucosal epithelium.

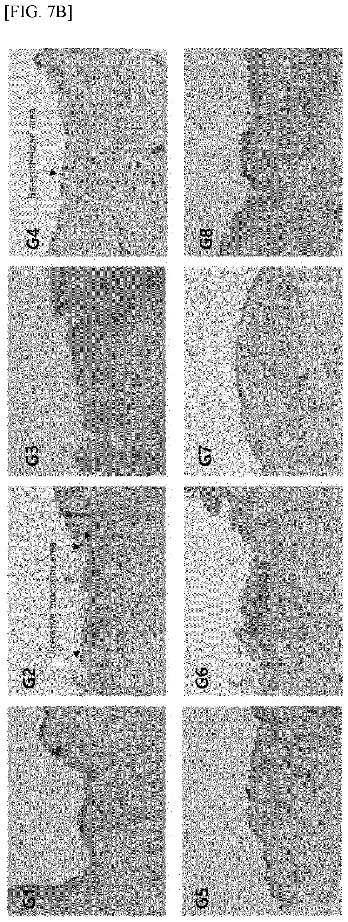

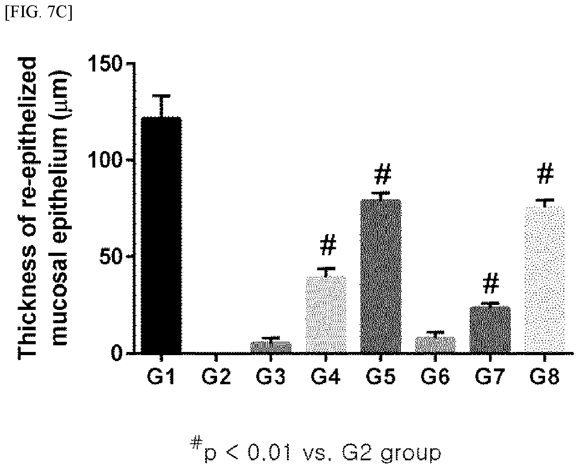

For G1, re-epithelialization was complete in all areas where ulcerative mucositis was induced, and slight tissue elevation due to proliferation of granulation tissue was observed in the submucosal area, wherein although some individuals still showed mild inflammatory findings, the majority had complete healing and showed normal structures (bottom left of FIG. 7B). For G2, re-epithelialization did not occur at any area where ulcerative mucositis was induced, and calcification, necrosis, proliferation of granulation tissue (infiltration of inflammatory cells) and the like were observed. For G3 and G6 (groups administered with SodA2 at low concentrations), no significant differences were observed compared to the G2 group, and areas of severe ulcerative mucositis were observed. On the other hand, for G4, G5, G7, and G8 (groups administered with SodA2 at medium and high concentrations), re-epithelialization occurred, leading to a decrease in ulcer size, and inflammation and necrosis in the submucosal area also tended to gradually decrease (FIG. 7B). The results obtained by quantitatively analyzing the degree of re-epithelialization showed a pattern similar to the previous graphs for scoring of oral mucositis. That is, the normal group G1 showed a high degree of re-epithelialization, and G2 showed nearly no re-epithelialization. For the drug administration group, re-epithelialization was promoted in a concentration-dependent manner; and for G4, G5, G7, and G8, which are the groups administered with SodA2 at high concentrations, re-epithelialization increased statistically significantly compared to G2 (FIG. 7C).

### Example 7. Scoring and histological evaluation of intestinal mucositis

### Scoring of intestinal mucositis

During the 10-day test period, the perianal area and feces were visually inspected once a day and the degree of diarrhea was evaluated using the scoring method shown in Table 8 below.

**[Table 8]**

| Score | Criteria |
|---|---|
| 0 | Normal feces |
| 1 | Slightly wet and soft feces indicating mild diarrhea |
| 2 | Moist and shapeless feces indicating moderate diarrhea |
| 3 | Loose feces indicating severe diarrhea |

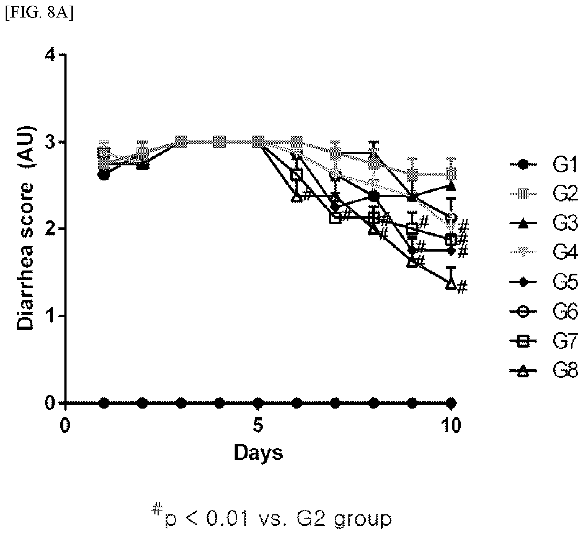

For G1, diarrhea did not occur during the 10-day test period. However, for the 5-FU administration groups (G2 to G8), diarrhea was observed from the start of drug administration; and these groups showed a pattern that severe, moist diarrhea persisted for the first 5 days of drug administration, and the diarrhea gradually improved over the next 5 days. In particular, for G4, G5, G7, and G8 which are groups administered with SodA2 at medium and high concentrations, the diarrhea score decreased statistically significantly compared to G2 (FIG. 8A).

### Histological evaluation of intestinal mucositis

Tissues containing the intestinal mucosa were collected, fixed in 10% formalin, and then subjected to standard histological processing to prepare hematoxylin and eosin (H&E)-stained slide glasses. Subsequently, the lesion site caused by mucositis was examined using a microscope to evaluate changes in intestinal villi in the mucositis area and analyze the intestinal villus length.

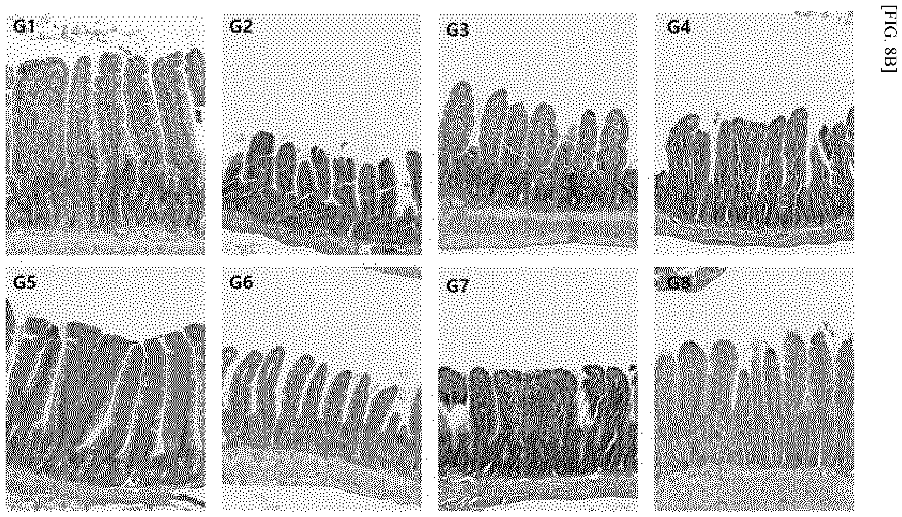

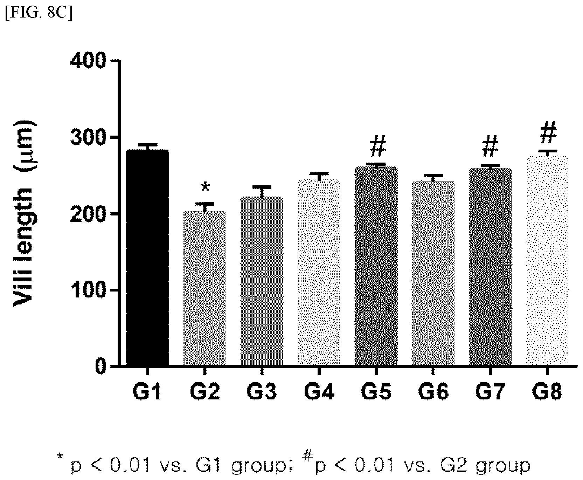

For G1, normal anatomical and histological structures of the small intestine, such as villi and crypts, were observed. On the other hand, for G2, severe villus atrophy was characteristically observed, which led to malabsorption, resulting in diarrhea and weight loss. For the drug administration groups (G3 to G8), improved villus atrophy was clearly observed in a concentration-dependent manner, and infiltration of inflammatory cells was mild and did not differ between the groups (FIG. 8B). The results obtained by quantitatively analyzing the intestinal villus length also showed that for G2, villus atrophy occurred compared to G1, resulting in decreased villus length. For the drug administration groups (G3 to G8), improved villus atrophy was clearly observed in a concentration-dependent manner compared to G2. In particular, it was found that for G5, G7, and G8, the intestinal villus length increased statistically significantly (FIG. 8C).

### Example 8. ROS evaluation of oral and intestinal mucositis

### ROS evaluation in small intestine

Autopsy was performed to collect intestinal tissue. Then, 100 mg of the intestinal tissue was washed twice with PBS, and 1 mL of ice-cold 50 mM TBS (pH 7.4) containing a mixture of protease inhibitors was added thereto. Then, the tissue was homogenized using a bead mill, and the homogenate was centrifuged at 12,000 rpm for 10 minutes at 4°C. The supernatant was collected and stored. The tissue homogenate was diluted with ice-cold Locke's buffer (154 mM NaCl, 5.6 mM KCl, 3.6 mM NaHCO₃, 2.0 mM CaCl₂, 10 mM D-glucose, and 5 mM HEPES, pH 7.4) to a final concentration of 5 mg tissue/mL. Subsequently, 0.2 mL of the homogenate was mixed with Locke's buffer (pH 7.4) and 10 µL of DCFH-DA (purchased from Sigma, 5 µM) to prepare a reaction mixture. 1 mL of the reaction mixture was incubated for 45 minutes, and the fluorescence of DCF was measured using a spectrofluorometer (Excitation: 484 nm, Emission: 530 nm). The blank was corrected using the background fluorescence of the homogenate that did not contain DCFH-DA.

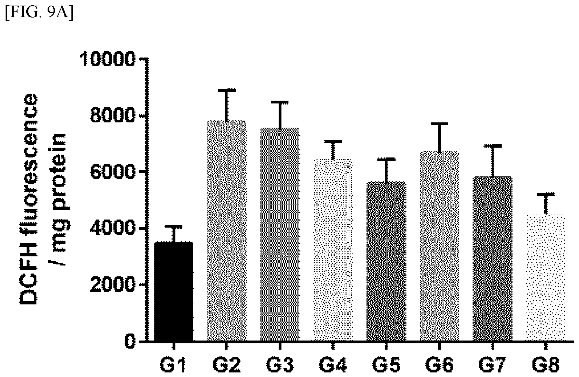

As a result of analyzing the ROS level in the small intestinal tissue through DCFH-DA, the ROS level of G2 showed an ROS level that was two-fold or more compared to G1. For the drug administration groups, both the oral application groups (G3 to G5) and the intragastric administration groups (G6 to G8) showed a concentration-dependent decrease compared to G2 (FIG. 9A). That is, it was confirmed that for 5-FU-induced mucositis, administration of SodA2 had an effect of decreasing the ROS level in a concentration-dependent manner in both the oral application groups and the intragastric administration groups.

### ROS evaluation in blood

ROS levels in the blood were measured using luminol-amplified chemiluminescence signals. 40 µL of plasma was dispensed into a 96-well plate, and then the reference value was measured using a chemiluminescence spectrometer. Then, 200 µL of luminol (purchased from Sigma, 200 mM, pH 7.4) was added to the 96-well plate, and then incubated for 10 minutes. The luminescence values were measured at 1-minute intervals and corrected using the reference value.

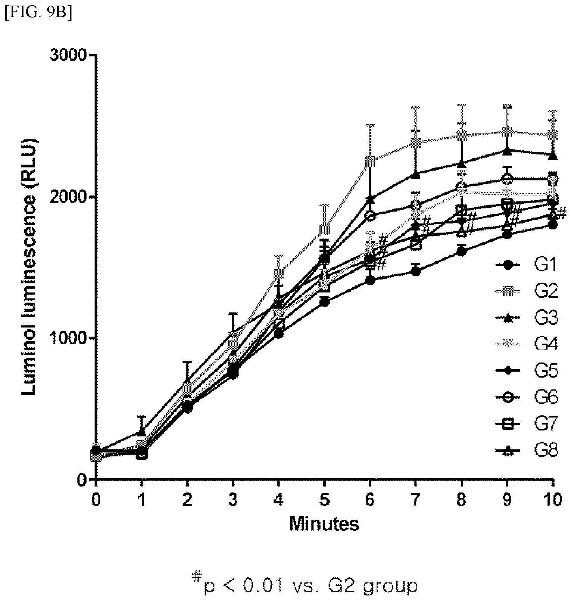

As a result of analyzing the ROS levels in the blood through luminol luminescence, G2 showed about a 0.5-fold increase in luminol luminescence compared to G1. For the drug administration groups, both the oral administration groups (G3 to G5) and intragastric administration groups (G6 to G8) showed a concentration-dependent decrease compared to G2. In particular, a statistically significant decrease was observed in G5, G7, and G8 at 6 minutes after luminol addition, in G5, G7, and G8 at 7 minutes after luminol addition, in G5 and G8 at 8 to 9 minutes after luminol addition, and in G8 at 10 minutes after luminol addition (FIG. 9B).

### Example 9. Statistical analysis

All numerical data were statistically analyzed using the GraphPad Prism statistical program (Ver. 8.0). First, Levene's test was performed to evaluate homogeneity of variance, and then one-way ANOVA test was performed to determine significance between the test groups. When the significance between the test groups was determined as a result of the one-way ANOVA, Tukey's post-hoc test was performed depending on the homogeneity of variance. All data were expressed as Mean ± SEM, and the confidence interval was set at 95%.

### Example 10. Conclusion and discussion

The anticancer agent 5-FU was intraperitoneally administered to induce both oral mucositis and intestinal mucositis, and then SodA2 was applied in the oral cavity and administered intragastrically (orally) for 10 days. As a result, it was found that SodA2 promoted re-epithelialization of the ulcer site at the oral mucosa and had efficacy of improving diarrhea and intestinal villus atrophy in a concentration-dependent manner. In addition, it was found that SodA2 decreased the ROS levels in the blood and intestinal tissue where mucositis occurred, thereby showing an effect of improving symptoms. It was confirmed that there was no significant difference due to the route of drug administration.

In conclusion, it was found that SodA2 (an SOD enzyme derived from Bacillus bacteria) can be a useful material for an agent that inhibits and treats oral and intestinal mucositis caused by side effects of chemotherapy.

## Claims

1. A pharmaceutical composition for treating or preventing mucositis, comprising a superoxide dismutase (SOD) derived from Bacillus as an active ingredient.

2. The pharmaceutical composition of claim 1, wherein the mucositis is caused by chemotherapy, radiotherapy, or a combination thereof.

3. The pharmaceutical composition of claim 1, wherein the mucositis comprises oral mucositis, esophageal mucositis, gastrointestinal mucositis, rectal mucositis, or a combination thereof.

4. The pharmaceutical composition of claim 1, wherein the SOD brings about at least one effect selected from reduced difficulty eating, ulcer healing, improved re-epithelialization, improved diarrhea symptoms, improved intestinal villus atrophy, and reduced reactive oxygen species (ROS) level in the gastrointestinal tract or blood.

5. The pharmaceutical composition of claim 1, wherein the composition is administered to a subject before, after, or both before and after receiving radiotherapy.

6. The pharmaceutical composition of claim 1, wherein the composition is administered to a subject before, after, or both before and after receiving chemotherapy.

7. The pharmaceutical composition of claim 1, wherein the composition is administered to a subject who is undergoing anticancer treatment.

8. The pharmaceutical composition of claim 6, wherein an anticancer agent used in the chemotherapy comprises at least one selected from cytotoxic anticancer agents, targeted anticancer agents, immune anticancer agents, or a combination thereof.

9. The pharmaceutical composition of claim 1, wherein the SOD is a Bacillus-derived Mn-SOD.

10. The pharmaceutical composition of claim 1, wherein the SOD comprises an amino acid sequence as set forth in SEQ ID NO: 4.

11. The pharmaceutical composition of claim 1, wherein the SOD is derived from *Bacillus amyloliquefaciens* GF423 strain (KCTC 13222BP).

12. The pharmaceutical composition of claim 1, wherein the SOD is an isolated or purified enzyme.

13. The pharmaceutical composition of claim 1, wherein the SOD is included in a form of a strain lysate, a strain culture, a strain culture concentrate, a strain culture extract, or a dried form thereof

14. The pharmaceutical composition of claim 1, wherein the SOD is applied in oral cavity or administered orally.

15. The pharmaceutical composition of claim 1, wherein the SOD is administered or applied at a dose of about 50 units/kg/day to about 2500 units/kg/day based on a subject's weight.

16. The pharmaceutical composition of claim 1, wherein the SOD is administered or applied at a dose of about 125 units/kg/day to about 1000 units/kg/day based on a subject's weight.

17. A veterinary composition for treating or preventing mucositis, comprising a superoxide dismutase (SOD) derived from Bacillus as an active ingredient.

18. A food composition for treating or preventing mucositis, comprising a superoxide dismutase (SOD) derived from Bacillus as an active ingredient.

19. A kit for treating or preventing mucositis, comprising the composition of any one of claims 1 to 18 and an instruction manual that comprises instructions for taking or administering the composition.

20. The kit of claim 19, wherein the instruction manual comprises instructions directing to use the composition in combination with radiotherapy and/or chemotherapy, or in combination with an anticancer agent.

21. A method for preventing or treating mucositis, comprising administering to a subject the composition of any one of claims 1 to 18 or a superoxide dismutase.

22. A use of the composition of any one of claims 1 to 18 or a superoxide dismutase, for preventing or treating mucositis.

23. A use of the composition of any one of claims 1 to 18 or a superoxide dismutase, in manufacture of a medicament for prevention or treatment of mucositis.
